# EUROPÄISCHE PATENTANMELDUNG

(11) **EP 1 820 527 A1**
(43) Veröffentlichungstag der Anmeldung: **22.08.2007**
(21) Anmeldenummer: 07403001.6
(22) Anmeldetag: 29.01.2007
(51) Int. Cl.: A61M 16/04

(54) **Vorrichtung zur endoskopischen Untersuchung des Schluckakts**

(30) Priorität: 21.02.2006 DE 102006008883; 13.07.2006 DE 102006033220
(71) Anmelder: ISKIA GmbH & Co. KG, 38829 Harsleben (DE)
(72) Erfinder: Neubauer, Norbert, 38820 Halberstadt (DE); Maltzahn, Meik, 38820 Halberstadt (DE); Riehl, Frank, 24119 Kronshagen (DE)
(74) Vertreter: Pfeiffer, Rolf-Gerd

(57) **Zusammenfassung**

Die Erfindung betrifft eine Vorrichtung zur endoskopischen Untersuchung des Schluckakts. Die Aufgabe der Erfindung, eine derartige Vorrichtung anzugeben, die eine Beobachtung auch genau im kurzen Moment des eigentlichen Schluckaktes ermöglicht, wird dadurch gelöst, dass eine geblockte Trachealkanüle (**1**) zum Einsatz gelangt, bei der im Krümmungsbereich (**10**) der Kunststoffkanüle (**11**), der dem Kehlkopfdeckel (**3**) zugewandt ist, eine Durchtrittsöffnung (**4**) vorgesehen ist, deren Berandung (**41**) trachealkanüleninwändig mit einem durch die Trachealkanüle nach außen geführten Arbeitskanal (**5**) zur Aufnahme und Führung eines biegsamen Endoskops verbunden ist, wobei der Arbeitskanal (**5**) bevorzugt gebildet ist durch einen Kanal, der einen Einführungsbereich (**52**) und einen im Bereich der Durchtrittsöffnung (**4**) liegenden Austrittbereich (**53**) für das Endoskop aufweist und welcher Bestandteil einer in die Kunststoffkanüle (**11**) lösbar einführbaren Innenkanüle (**9**) ist.

## Beschreibung

Die Erfindung betrifft eine Vorrichtung zur endoskopischen Untersuchung des Schluckakts, die im Falle ausgewählter Patienten mit neurogener Dysphagie zum Einsatz gelangen kann. In der Medizin sind vielfältige Ursachen zur Auslösung einer neurogenen Dysphagie bekannt und die bekannten Untersuchungsmethoden weisen nachstehend beschriebene Vor- respektive Nachteile auf.

Die zwei wichtigsten grundsätzlichen Untersuchungsmethoden des Schluckakts sind die Schluckendoskopie und die Videofluoroskopie. Während bei letzterer der gesamte Schluckablauf des Patienten, insbesondere während des kurzen Schluckvorgangs selbst verfolgt werden kann, hat diese Methode den grundsätzlichen Nachteil einer Strahlenbelastung und kann nur beim wachen Patienten durchgeführt werden.
Die Schluckendoskopie wird nach dem bekannten Stand der Technik vermittels eines biegsames Endoskops, das über die Nase eingeführt wird, vorgenommen. Damit sind Ansichten der Zunge, der Rachenwand sowie der Stimmlippen und evtl. auch der Luftröhre möglich. Die Videoaufzeichnung derart gewonnener Ansichten gehört seit längerem zum bekannten Stand der Technik. Der Hauptvorteil dieser Methode besteht darin, dass außer des Wegfalls der Strahlenbelastung nach vorstehend skizzierter Methode, hier Untersuchungen auch beim nichtkooperativen, liegenden oder sogar bewusstlosen Patienten vorgenommen werden können. Im Gegensatz zur zuerst erwähnten Methode besteht der Hauptnachteil bei der bekannten transnasalen Endoskopie jedoch darin, dass genau in dem eigentlich interessanten Moment, nämlich dem des Schluckaktes, die Sicht durch die plötzliche Kontraktion der Schluckmuskulatur für das Endoskop versperrt wird.

Der Erfindung liegt daher die Aufgabe zugrunde, eine Vorrichtung zur endoskopischen Untersuchung des Schluckakts anzugeben, die eine Beobachtung auch genau im kurzen Moment des eigentlichen Schluckaktes ermöglicht und in weiterer Ausgestaltung der Erfindung, technologisch einfach zu realisieren ist.
Die vorgeschlagene Vorrichtung kommt insbesondere bei Patienten zum Einsatz, ist jedoch nicht darauf beschränkt, die bereits eine Trachealkanüle tragen müssen. Diese Patienten, die zusätzlich von einer Dysphagie betroffen sein können, gelingt tlw. nicht einmal mehr das sichere Abschlucken des Speichels. Bei diesen Patienten wird als Hilfsmittel, um zu verhindern, dass Speichel in die Luftröhre und damit die Lungen gelangt, eine so genannte geblockte Trachealkanüle eingesetzt. Dabei wird nach operativer Eröffnung der Luftröhre ein gebogenes Kunststoffrohr in die Luftröhre eingeführt, wobei das in der Luftröhre liegende Ende mit einem aufblasbarem Ballon versehen ist, der ein Abdichten gegen die Luftröhre bewirkt und damit ein Eindringen von Speichel in die Luftröhre verhindert. Sich eventuell über dem Ballon zwischen Luftröhre und Trachealkanüle ansammelnder Speichel kann über eine gesondert verlegte weitere Kanülen abgesaugt werden.

Die Aufgabe der Erfindung wird unter Verwendung einer an sich bekannten geblockten Trachealkanüle durch die Merkmale von Anspruch 1 gelöst. Vorteilhafte Ausgestaltungen sind Gegenstand der nachgeordneten Ansprüche.

Das Wesen der Erfindung besteht darin, dass eine geblockte Trachealkanüle zum Einsatz gelangt, in deren Krümmungsbereich, der dem Kehlkopfdeckel zugewandt ist, eine Durchtrittsöffnung vorgesehen ist, deren Berandung trachealkanüleninwändig mit einem durch die Trachelkanüle nach außen (das heißt, aus der Eintrittsöffnung hervorragenden) geführten Arbeitskanal zur Aufnahme und Führung eines biegsamen Endoskops dicht verbunden ist. In weiterer Ausgestaltung der Erfindung ist genannter Arbeitskanal gebildet durch einen Kanal, der einen Einführungsbereich und einen im Bereich der Durchtrittsöffnung liegenden Austrittbereich für ein Endoskop aufweist und welcher Bestandteil einer in die Kunststoffkanüle lösbar einführbaren Innenkanüle ist.

Die Erfindung soll nachstehend anhand eines Ausführungsbeispiels und schematischer Zeichnungen näher erläutert werden. Es zeigen:
- Fig. 1: eine mögliche erste Ausführungsform der Erfindung in geschnittener Seitenansicht,
- Fig. 2, 2a und 2b: Detaildarstellungen und alternative Ausführungen der ersten erfindungsgemäßen Vorrichtung,
- Fig. 3: eine bevorzugte zweite Ausführungsform der Erfindung in geschnittener Seitenansicht mit schematisch angedeutetem anatomischen Umfeld (analog Fig. 1),
- Fig. 4: einen detaillierteren Längsschnitt durch eine zum Einsatz gelangende Innenkanüle nach Fig. 3,
- Fig. 5: einen Querschnitt entlang einer Ebene A-A gemäß Fig. 4,
- Fig. 6: eine Teildraufsicht auf eine Innenkanüle nach Fig. 4 und
- Fig. 7: eine Teildraufsicht auf eine Kunststoffkanüle mit einer möglichen besonderen Ausbildung einer Durchtrittsöffnung nach Fig. 3.

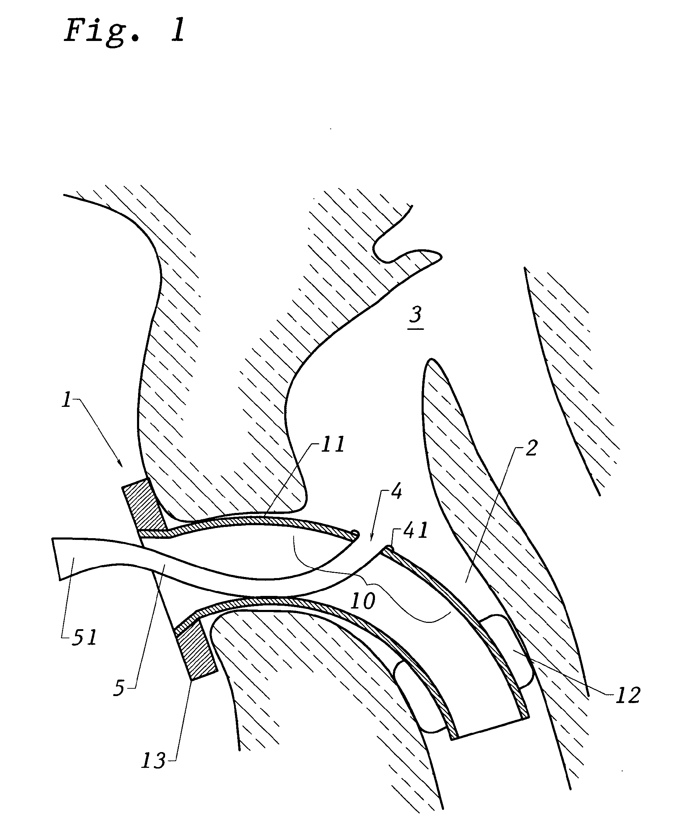

In Figur 1 ist eine geblockte Trachealkanüle **1,** im Wesentlichen bestehend aus einer in die Luftröhre **2** eingeführten, gekrümmten Kunststoffröhre **11,** einem außen am Hals des im entsprechenden Bereich schematisch dargestellten Patienten am Stoma anliegenden Flansch **13** und einem aufblasbaren, mit der gekrümmten Kunststoffröhre **11** verbundenen Ballon (cuff) **12,** dargestellt. Die in Figur 1 nicht dargestellten zusätzlichen Kanäle zum Aufblasen des Ballons **12** und zum Absaugen von oberhalb des Ballons zwischen Luftröhre **2** und der Kunststoffröhre **11** angesammelten Speichels, sind dabei bevorzugt bei Extrusion der Kunststoffröhre **11** innerhalb deren Wandung eingebracht. In Figur 1 ist weiterhin im seitlichen Schnitt im Krümmungsbereich **10** der Trachealkanlüle, der dem Kehlkopfdeckel **3** zugewandt ist, eine Durchtrittsöffnung **4** innerhalb der Trachealkanülenwandung vorgesehen. Diese Durchtrittsöffnung **4** ist mit einem innerhalb der Trachealkanüle angeordneten Arbeitskanal **5** im Bereich der Berandung **41** der Durchtrittsöffnung **4** dicht und vorzugsweise unlösbar verbunden. Figur 1 zeigt schematisch, dass zumindest die luftröhrenseitigen Bereiche der Berandung **41** vorzugsweise atraumatisch geformt sind, was durch eine thermische Anformung des distalen Endes des Arbeitskanals **5** bewerkstelligt werden kann. In Figur 1 ist die atraumatische Berandungsbereichsausbildung schematisch wulstförmig angedeutet. Andere Befestigungsmöglichkeiten des Arbeitskanals **5** mit der Trachealkanülenwandung, bspw. durch eine trachealkanüleninwändige Verklebung oder Verschweißung, liegen selbstverständlich im Rahmen der Erfindung. Weiterhin ist genannter Arbeitskanal **5** an seinem nach außen ragenden und den Flansch **13** überragenden Ende in Form eines Trichters **51** ausgebildet. Diese trichterförmige Erweiterung dient als Einführhilfe für ein nicht dargestelltes flexibles Endoskop. Dieses wird im Falle der Untersuchung des Schluckvorgangs in den Arbeitskanal **5** eingeführt und gelangt zwangsgeführt durch die spezielle Krümmungsausbildung des Arbeitskanals **5** im Bereich der Durchtrittsöffnung **4,** in dorsale Lage zum Kehlkopfdeckel **3.**

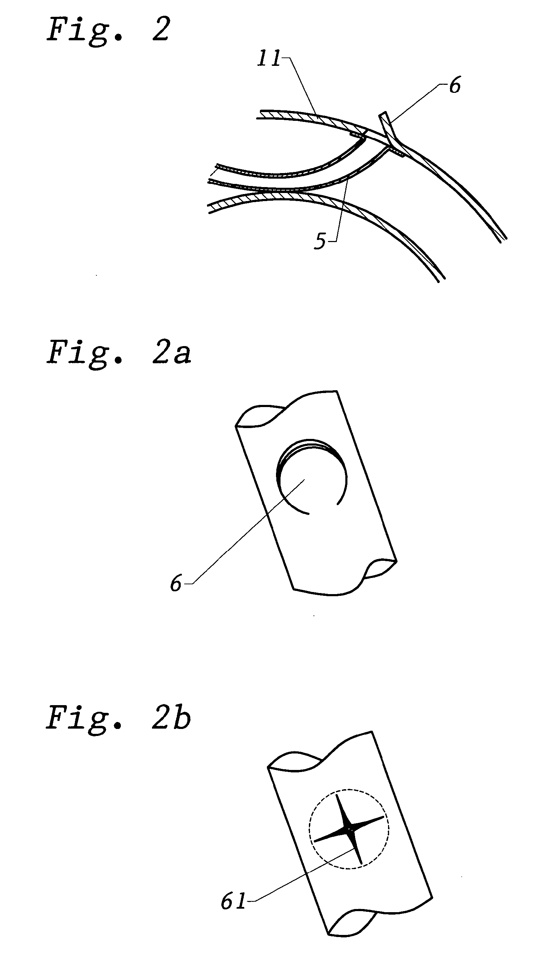

In weiterer Ausgestaltung der Erfindung, bspw. wie in den Figuren 2, 2a und 2b in je einer Ausschnittsdarstellung angedeutet, ist es vorteilhaft, den Bereich der Durchtrittsöffnung **4** mit einer Klappe **6** zu versehen, um ein sicheres Einführen der Trachealkanüle **1** in die Luftröhre **2** zu ermöglichen und dabei ein Verunreinigen des Arbeitskanals **5** zu verhindern.
Dies kann im Rahmen der Erfindung auf vielfältige Weise erfolgen. In Abhängigkeit von der Wandstärke der Kunststoffröhre **11** im gekrümmten Bereich **10** und der Elastizität des für die Trachealkanüle eingesetzten Materials kann bspw. die Einbringung eines Mehrfachschlitzes, insbesondere eines Kreuzschlitzes **61** in die Kanülenwandung (vgl. Fig. 2b) einen klappenartig abgedeckten und dennoch den Durchtritt des Endoskops ermöglichenden Bereich bilden. Ebenso kann durch einen kreisförmigen Einschnitt in die Kanülenwandung selbst eine derartige Klappe **6** gebildet sein, wie es in den Figuren 2 und 2a aus verschiedenen Sichten angedeutet ist. Bei beiden vorstehend beschriebenen Ausführungen erfolgt die Anbindung des distalen Endes des Arbeitskanals **5** inwändig an die Trachealkanüle. So es die Materialeigenschaften der Trachealkanüle erforderlich machen sollten, kann die Wandstärke der Trachealkanüle während ihrer Herstellung im Bereich der Durchtrittsöffnung auch dünner als die übrigen Wandungsbereiche der Kunststoffröhre **11** ausgebildet sein.
Eine aufwändigere, aber ebenfalls im Rahmen der Erfindung liegende Lösung zur Ausbildung einer Klappe **6** ist deren gesonderte nachträgliche Anbringung und gelenkige Anbindung an die Wandung der Trachealkanüle im Bereich der Durchtrittsöffnung **4,** was hier jedoch nicht gesondert dargestellt ist.

Die Einbindung des Arbeitskanals **5** an die Durchtrittsöffnung **4** sollte in jedem Fall derart erfolgen, das die Tangente am Arbeitskanal **5** im Bereich der Eintrittsöffnung mit der Tangenten an der Trachealkanüle 11 in eben diesem Bereich bevorzugt einen Winkel in der Größenordnung von 90° einschließt. Im Rahmen der Gegebenheiten, da alle bekannten Trachealkanülen im wesentlichen über den gleichen Krümmungsradius verfügen, kann dies durch die in Figur 1 geschwungen dargestellte Verlegung des Arbeitskanals **5** weitestgehend realisiert werden.

In jedem Fall der Ausführung der in diesem Beispiel vorgeschlagenen Vorrichtung ist jedoch zu beachten, dass der äußere Durchmesser des Arbeitskanals **5** kleiner oder höchsten in der Größenordnung der Hälften des Innendurchmessers der Kunststoffröhre **11** festgelegt ist, um einen nicht unnötig hohen Beatmungswiderstand für den Patienten zu gewährleisten.

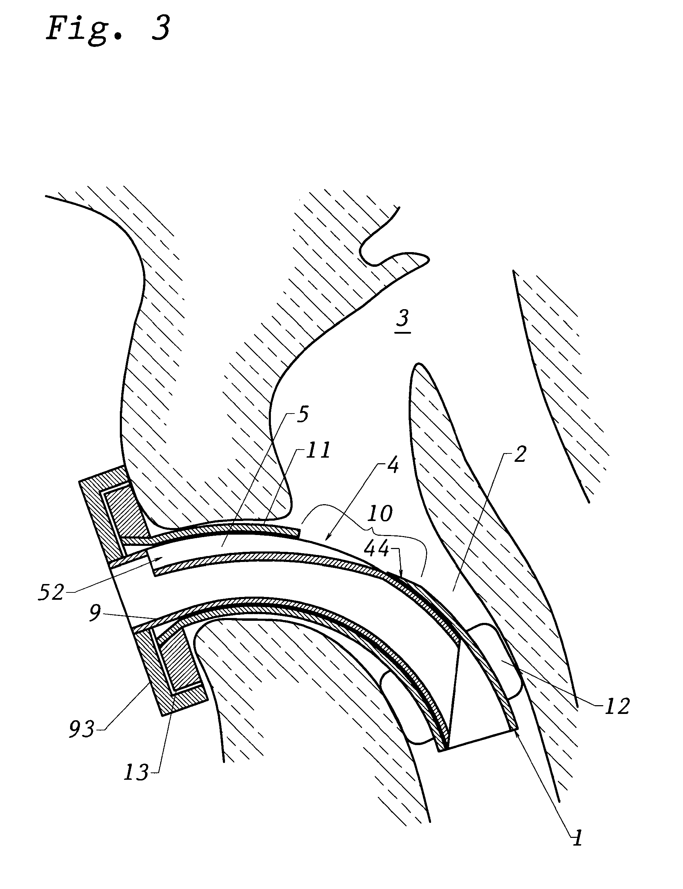

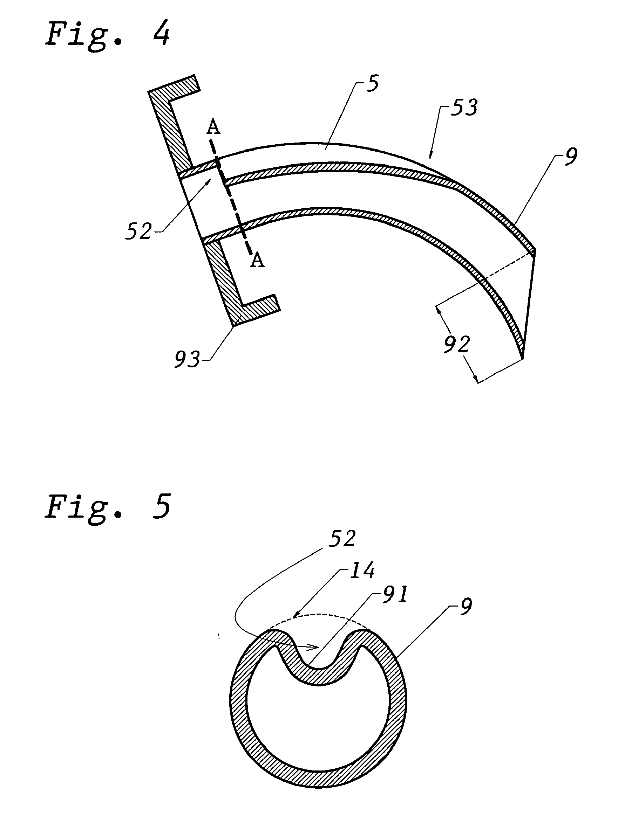

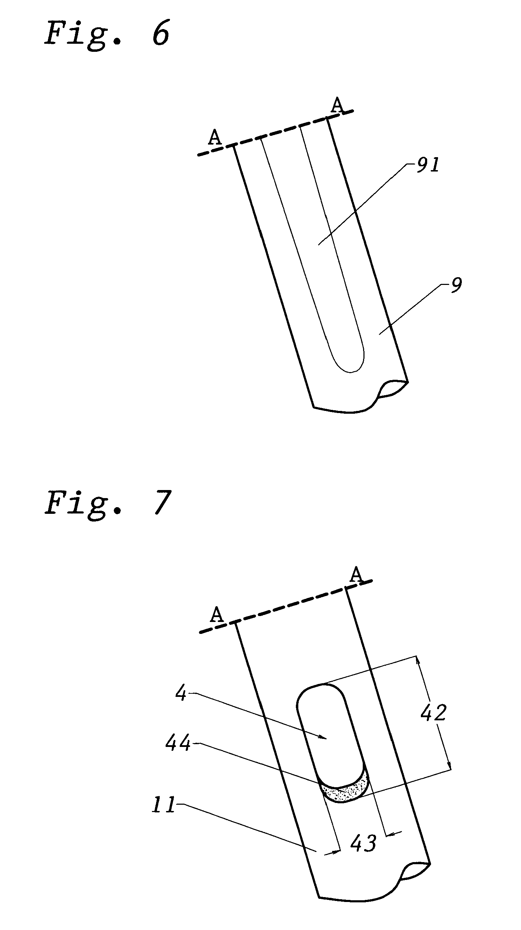

Figur 3 zeigt eine bevorzugte, weil technologisch einfacher herzustellende, zweite Ausführungsform der Erfindung. Hier ist ebenfalls eine geblockte Trachealkanüle **1,** im Wesentlichen bestehend aus einer in die Luftröhre **2** eingeführten, gekrümmten Kunststoffröhre **11,** einem außen am Hals des im entsprechenden Bereich schematisch dargestellten Patienten am Stoma anliegenden Flansch **13** und einem aufblasbaren, mit der gekrümmten Kunststoffröhre **11** verbundenen Ballon (cuff) **12,** dargestellt. Bezüglich der auch hier nicht dargestellten zusätzlichen Kanäle zum Aufblasen des Ballons **12** und zum Absaugen von oberhalb des Ballons zwischen Luftröhre **2** und der Kunststoffröhre **11** angesammelten Speichels, sind die gleichen Maßnahmen, wie nach Fig. 1 beschrieben, vorgesehen. In Fig. 3 ist weiterhin im seitlichen Schnitt im Krümmungsbereich **10** der Trachealkanlüle, der dem Kehlkopfdeckel **3** zugewandt ist, eine Durchtrittsöffnung **4** innerhalb der Trachealkanülenwandung vorgesehen. Diese Durchtrittsöffnung **4** steht mit einem innerhalb der Trachealkanüle angeordneten Arbeitskanal **5** in Verbindung. Gemäß dieses Ausführungsbeispiels ist genannter Arbeitskanal **5** gebildet durch einen Kanal, der einen Einführungsbereich **52** und einen im Bereich der Durchtrittsöffnung **4** liegenden Austrittbereich **53** (vgl. Fig. 4) für ein nicht dargestelltes Endoskop aufweist, und welcher Bestandteil einer in die äußere Kunststoffkanüle **11** lösbar einführbaren Innenkanüle **9** ist, wie sie in Figur 4 detaillierter dargestellt ist.
Zur weiteren Verdeutlichung der Ausbildung der vorgeschlagenen Innenkanüle **9** zeigt Figur 5 in einem Schnitt entlang einer Ebene A-A nach Fig. 4 die spezielle Gestaltung des Arbeitskanals, der durch eine Nut **91,** die in den Außenumfang in Längsrichtung der Innenkanüle **9** eingebracht ist, gebildet ist, wobei der Kanalabschluss **14** an der offenen Nutseite durch die Innenwand der Kunststoffkanüle **11** im eingebauten Zustand der Innenkanüle **9** gebildet ist, wie es in Fig. 5 durch den strichlinierten oberen Bogen angedeutet ist. In Längsrichtung der Innenkanüle **9** gesehen, ist die Nut **91** im weiteren so ausgebildet, dass sie im vollständig montierten Zustand in die Kunststoffkanüle **11** im Bereich der Durchtrittsöffnung **4** der Kunststoffkanüle **11** endet. Bevorzugt ist die Nut **91** dabei so ausgeführt, dass sie im Auslaufbereich der Durchtrittsöffnung **4** keilförmig oder verrundet zulaufend endet und in der Tiefe verjüngt ausgeführt ist. In der Teildraufsicht nach Figur 6 ist die vorstehend beschriebene Ausbildung der Nut **91** ersichtlich und die Verjüngung ist aus Fig. 3 und Fig. 4 im seitlichen Schnitt ersichtlich.
Selbstverständlich liegt es auch im Rahmen dieses Ausführungsbeispiels, die Durchtrittsöffnung **4** mit einer Klappe **6** oder einem Kreuzschlitz **61** zu versehen, wie in den Figuren 2a und 2b dargestellt.

In weiterer besonderer Ausgestaltung ist vorgesehen, dass die Längserstreckung **42** der Durchtrittsöffnung **4** der Kunststoffkanüle **11** größer als deren Breiteneröffnung **43** festgelegt ist und insbesondere das 1,5-Fache der Breite beträgt, was Figur 7 in einer Teildraufsicht schematisch verdeutlicht. Ebenfalls ist Fig. 7 entnehmbar, dass die Berandung der Durchtrittsöffnung **4** insbesondere oval bis elliptisch ausgeführt sein soll, um weitestgehend stetige Übergänge zu realisieren. Dabei liegt es selbstverständlich im Rahmen der Erfindung, dass die Berandung als solche atraumatisch ausgeformt ist, was nicht näher dargestellt ist, um den Patienten möglichst wenig zu belasten.

Weiterhin zeigt Fig. 7 im Zusammenhang mit Fig. 3, dass die Berandung der Durchtrittsöffnung **4** zumindest in einem Bereich **44** des Übergangs zur keilförmig ausgebildeten Nut **91** auf diese zulaufend konisch verjüngt ausgebildet ist. Dadurch ergibt sich eine problemlose Führung des in den Einführungsbereich **52** eingeführten und im Austrittbereich **53** austretenden nicht dargestellten Endoskops. Insbesondere ergibt sich durch die vorstehend beschriebenen speziellen Ausbildungen ein größeres Spiel für die Endoskopführung, weil dadurch ein großes Positionierfeld für den Endoskopkopf geschaffen ist, wodurch anatomische Besonderheiten des Patienten leichter berücksichtigt werden können und keine Fixlagen des Endoskopkopfes einschränkend auf die Verwendung der vorgeschlagenen Vorrichtung wirken.

Weiterhin vorteilhaft an der im bevorzugten Ausführungsbeispiel vorgeschlagenen Vorrichtung ist, dass sich das Endoskop bei entnommener Innenkanüle **9** ohne besondere Einführhilfen bereits in den Einführbereich **52** teilweise einlegen lässt, woran anschließend die Innenkanüle **9** in die bereits im Patienten verlegte Kunststoffkanüle **11** eingeführt wird und anschließend vermittels eines komplementär ausgebildeten Flansches **93** an der Innenkanüle **9** mit einem Flansch **13** als Gegenstück an der Kunststoffkanüle **11** rastend oder klemmend in Eingriff gebracht wird. Danach erfolgt die vollständige Einbringung und Feinpositionierung des Endoskops im Arbeitskanal **5** und aus der Durchtrittsöffnung **4.**

Die nach Fig. 3 vorgeschlagene Innenkanüle gewährleistet für den Patienten ebenfalls einen möglichst geringen Beatmungswiderstand. Weiterhin ist durch die problemlose Entnehmbarkeit der Innenkanüle **9** und deren konstruktiv einfachen Aufbau und allseitigen Zugang eine unkomplizierte Reinigungsmöglichkeit aller Komponenten einschließlich des Arbeitskanals **5** gegeben. Letztere beschriebenen Vorteile zeichnen die Ausführung nach Fig. 3 als vorteilhafter gegenüber einer Ausführung nach Fig. 1 aus.

Die Feinausrichtungen zur genauen Positionierung des Eintrittsfensters des nicht dargestellten Endoskops in dorsale Lage zum Kehlkopfdeckel 3 erfolgt dann in beiden Ausführungsformen der Erfindung bspw. über übliche Zugmechanismen, die aber Gegenstand des Endoskops selber und nicht vorliegender Erfindung sind.

Durch die vorgeschlagene Vorrichtung in zwei Ausführungsvarianten wird eine neuartige endoskopische Diagnosemöglichkeit geschaffen, die nicht die Nachteile bekannter vergleichbarer transnasaler Endoskopieverfahren aufweist, da mit Hilfe der vorgeschlagenen Vorrichtungen, insbesondere durch die dorsale Betrachtung des Laryngs gerade im Moment des Schluckvorgangs gewährleistet ist und beobachtet werden kann, wie beim Schlucken Speichel oder Speisen den Kehlkopf in Richtung der Trachea passieren, was bei Beibehaltung der oben beschriebenen Vorteile endoskopischer Untersuchungsverfahren erstmals eine sichere Schluckdiagnose vermittels flexibler Endoskope ermöglicht.

Es liegt im Rahmen anderer durch den behandelnden Arzt zu erbringender Diagnosen, ob die vorstehend beschriebene Vorrichtung auch dann zum Einsatz gelangen kann oder muss, wenn der Patient noch nicht über ein Stoma in der Trachea verfügt.

Alle in der Beschreibung, den Ausführungsbeispielen und den nachfolgenden Zeichnungen erkennbaren Merkmale können sowohl einzeln als auch in beliebiger Kombination miteinander erfindungswesentlich sein.

### Bezugszeichenliste

- 1 -: geblockte Trachealkanüle
- 10 -: gekrümmter Bereich der Kunststoffkanüle
- 11 -: Kunststoffkanüle
- 12 -: Ballon (cuff)
- 13 -: Flansch
- 2 -: Luftröhre
- 3 -: Kehlkopfdeckel
- 4 -: Durchtrittsöffnung
- 41 -: Berandung der Durchtrittsöffnung
- 42 -: Längserstreckung der Durchtrittsöffnung 4
- 43 -: Breite der Durchtrittsöffnung 4
- 44 -: Übergangsbereich der Durchtrittsöffnung 4
- 5 -: Arbeitskanal
- 51 -: Trichter
- 52 -: (Endoskop)-Einführungsbereich
- 53 -: (Endoskop)-Austrittsbereich
- 6 -: Klappe
- 61 -: Kreuzschlitz
- 9 -: Innenkanüle
- 91 -: Nut
- 92 -: Endbereich der Innenkanüle 9
- 93 -: Flansch an der Innenkanüle 9
- A-A -: Schnitt

## Patentansprüche

1. Vorrichtung zur endoskopischen Untersuchung des Schluckakts, wobei eine geblockte Trachealkanüle **(1)** zum Einsatz gelangt, bei der im Krümmungsbereich **(10)** der Kunststoffkanüle **(11),** der dem Kehlkopfdeckel **(3)** zugewandt ist, eine Durchtrittsöffnung **(4)** vorgesehen ist, deren Berandung **(41)** trachealkanüleninwändig mit einem durch die Trachelkanüle nach außen geführten Arbeitskanal **(5)** zur Aufnahme und Führung eines biegsamen Endoskops verbunden ist.

2. Vorrichtung nach Anspruch 1, **dadurch gekennzeichnet, dass** die Berandung **(41)** der Durchtrittsöffnung **(4)** zumindest luftröhrenseitig atraumatisch geformt ist.

3. Vorrichtung nach Anspruch 1 oder 2, **dadurch gekennzeichnet, dass** die Berandung **(41)** der Durchtrittsöffnung **(4)** mit dem distalen Ende des Arbeitskanals **(5)** thermisch verscheißt ist.

4. Vorrichtung nach Anspruch 1 oder 2, **dadurch gekennzeichnet, dass** die Berandung **(41)** der Durchtrittsöffnung **(4)** mit dem distalen Ende des Arbeitskanals **(5)** verklebt ist.

5. Vorrichtung nach Anspruch 1, **dadurch gekennzeichnet, dass** das proximale Ende des Arbeitskanals **(5)** in Form eines Trichters **(51)** erweitert ausgebildet oder angeformt ist.

6. Vorrichtung nach Anspruch 1, **dadurch gekennzeichnet, dass** die Durchtrittsöffnung **(4)** mit einer Klappe **(6)** versehen ist.

7. Vorrichtung nach Anspruch 6, **dadurch gekennzeichnet, dass** die Klappe **(6)** durch eine einseitig an die Trachealkanülenwandung **(11)** angelenkte und sich im übrigen an die Trachealkanüle anschmiegenden Klappe gebildet ist.

8. Vorrichtung nach Anspruch 6, **dadurch gekennzeichnet, dass** die Klappe **(6)** durch einen teilweisen, im wesentlichen kreisförmigen Einschnitt in die Trachealkanülenwandung **(11)** gebildet ist.

9. Vorrichtung nach Anspruch 6, **dadurch gekennzeichnet, dass** die Klappe **(6)** durch eine umfangsseitig mit der Berandung **(41)** verbundenen elastischen Abdeckung gebildet ist, die in ihrem Mittenbereich einen Kreuzschlitz **(61)** aufweist.

10. Vorrichtung nach Anspruch 1 und 6, **dadurch gekennzeichnet, dass** die Berandung **(41)** trachealkanüleninwändig mit dem distalen Ende des Arbeitkanals **(5)** verbunden ist und die Durchtrittsöffnung **(4)** durch einen in die Wandung der Trachealkanüle **(11)** in diesem Bereich eingebrachten durchgehenden Kreuzschlitz **(61)** gebildet ist.

11. Vorrichtung nach Anspruch 6 oder 10, **dadurch gekennzeichnet, dass** die Wandungsstärke der Trachealkanüle **(11)** im Bereich der Durchtrittsöffnung **(4)** dünner als in den übrigen Wandungsbereichen der Trachealkanüle ausgeführt ist.

12. Vorrichtung nach Anspruch 1, **dadurch gekennzeichnet, dass** der äußere Durchmesser des Arbeitskanals **(5)** kleiner als die Hälfte des Innendurchmessers der Kunststoffröhre **(11)** festgelegt ist.

13. Vorrichtung nach Anspruch 1, **dadurch gekennzeichnet, dass** die Tangente am Arbeitskanal **(5)** im Bereich der Durchtrittsöffnung **(4)** mit der Tangenten an der Trachealkanüle in eben diesem Bereich bevorzugt einen Winkel in der Größenordnung von 90° einschließt, wobei der Arbeitskanal **(5)** geschwungen verlegt an das Kunststoffrohr **(11)** angebunden ist.

14. Vorrichtung nach Anspruch 1, **dadurch gekennzeichnet, dass** die Durchtrittsöffnung **(4),** die trachealkanüleninwändig mit einem durch die Trachelkanüle nach außen geführten Arbeitskanal **(5)** zur Aufnahme und Führung eines biegsamen Endoskops in Verbindung steht, wobei der Arbeitskanal **(5)** gebildet ist durch einen Kanal, der einen Einführungsbereich **(52)** und einen im Bereich der Durchtrittsöffnung **(4)** liegenden Austrittbereich **(53)** für das Endoskop aufweist und welcher Bestandteil einer in die Kunststoffkanüle **(11)** lösbar einführbaren Innenkanüle **(9)** ist.

15. Vorrichtung nach Anspruch 14, **dadurch gekennzeichnet, dass** der Kanal **(5)** gebildet ist durch eine Nut **(91),** die in den Außenumfang in Längsrichtung der Innenkanüle **(9)** eingebracht ist, wobei der Kanalabschluss **(14)** an der offenen Nutseite durch die Innenwand der Kunststoffkanüle **(11)** gebildet ist.

16. Vorrichtung nach Anspruch 14 und 15, **dadurch gekennzeichnet, dass** die Nut **(91)** so lang ausgeführt ist, dass sie im Bereich der Durchtrittsöffnung **(4)** der Kunststoffkanüle **(11)** im vollständig eingesetzten Zustand endet.

17. Vorrichtung nach Anspruch 16, **dadurch gekennzeichnet, dass** Nut **(91)** im Auslaufbereich der Durchtrittsöffnung **(4)** keilförmig oder verrundet zulaufend und in der Tiefe sich stetig verjüngend ausgeführt ist.

18. Vorrichtung nach Anspruch 14, **dadurch gekennzeichnet, dass** der Durchtrittsöffnung **(4)** eine größere Längserstreckung **(42)** als Breite **(43)** gegeben ist.

19. Vorrichtung nach Anspruch 18, **dadurch gekennzeichnet, dass** die Längserstreckung **(42)** der Durchtrittsöffnung **(4)** größer als deren Breiteneröffnung **(43)** festgelegt ist und insbesondere das 1,5-Fache der Breite beträgt.

20. Vorrichtung nach Anspruch 18 und 19, **dadurch gekennzeichnet, dass** die Berandung der Durchtrittsöffnung **(4)** oval bis elliptisch ausgeführt ist.

21. Vorrichtung nach Anspruch 17 und einem der Ansprüche 18, 19 oder 20, **dadurch gekennzeichnet, dass** die Berandung der Durchtrittsöffnung **(4)** zumindest in einem Bereich **(44)** des Übergangs zur keilförmig ausgebildeten Nut **(91)** auf diese zulaufend konisch verjüngt ausgebildet ist.

22. Vorrichtung nach Anspruch 14, **dadurch gekennzeichnet, dass** die im wesentlichen rohrförmig ausgebildete Innenkanüle **(9)** in ihrem Endbereich **(92)** derart abgeschrägt ausgebildet ist, dass sie bei Einführung in die Kunststoffkanüle **(11)** eine gleitende und abstoßende Vorbeiführung an der Durchtrittsöffnung **(4)** gewährleistet.

23. Vorrichtung nach Anspruch 14, **dadurch gekennzeichnet, dass** die Innenkanüle **(9)** mit einem komplementär ausgebildeten Flansch **(93)** versehen ist, der in einen Flansch **(13)** der Kunststoffkanüle **(11)** rastend oder klemmend eingreift.
